# EUROPEAN PATENT APPLICATION

(11) **EP 3 985 121 A1**
(43) Date of publication of application: **20.04.2022**
(21) Application number: 20201956.8
(22) Date of filing: 15.10.2020
(51) Int. Cl.: C12P 17/04, C12P 41/00

(54) **ENANTIOSELECTIVE PRODUCTION OF (R)-PANTOLACTONE**

(71) Applicant: DSM IP Assets B.V., 6411 TE Heerlen (NL)
(72) Inventor: MEDLOCK, jonathan Alan, 4303 Kaiseraugst (CH); BONRATH, Werner, 4303 Kaiseraugst (CH); MÜLLER, Monika, 6167 RD Geleen (NL); VERMOTE, Linda Andrea Laura, 6167 RD Geleen (NL)
(74) Representative: Seibel-Thomsen, Nadja

(57) **Abstract**

The present invention relates to formation of highly enriched (R)-pantolactone from conversion of keto-pantolactone in a bio-catalytic process using enantioselective enzymes at low pH.

## Description

The present invention relates to formation of highly enriched (R)-pantolactone from conversion of keto-pantolactone in a bio-catalytic process at low pH.

Pantothenate or vitamin B5 is a member of the B complex of vitamins which is essential for mammals including humans. It has to be supplied as food or feed additive. In cells, pantothenate is used primarily for the biosynthesis of coenzyme A and acyl carrier protein. These essential coenzymes function in the metabolism of acyl moieties, which form thioesters with the sulfhydryl group of the 4'-phosphopantethein portion of these molecules. Importantly, only (R)-pantothenate is biologically active as vitamin.

Pantothenate has been synthesized conventionally via chemical synthesis from bulk chemicals. However, the substrates required for chemical synthesis are expensive and the racemic intermediates have to be optically resolved. Bacterial or microbial systems, if available, that produce enzymes useful in pantothenate biosynthesis processes, are not yet feasible for industrial production because of insufficient yield and/or selectivity of the used enzymes.

Thus, there is a need for more effective and simpler methods towards enantioselective production of (R)-pantolactone with a high enantiomeric excess (e.e.) to be used in an industrial scale, with no need for further resolution and/or purification steps.

Surprisingly, we now identified a novel process for production of (R)-pantolactone via enantioselective catalytic reduction of keto-pantolactone (KPL), comprising the use of selective reductases at a pH below 7.0.

Particularly, the present invention is directed to enantioselective reduction of KPL into (R)-pantolactone, wherein the reduction of KPL is catalyzed by enantioselective alcohol dehydrogenase (ADH) enzymes at a pH below 7.0, wherein an e.e. in the range of about 80-99% could be achieved.

For the purpose of the present invention, i.e. enantioselective conversion of KPL into (R)-pantolactone as described herein, any enzyme of class [EC 1.1.1.2] isolated from various sources can be used, particularly such enzymes isolated from archaea, bacteria, fungi including yeast, or higher eukaryotes. Preferably, the enzyme is selected from yeast, such as Candida or Saccharomyces, or Bacillus, such as Stenotrophomonas, preferably a NAD(P)H-dependent reductase selected from *Candida parapsilosis, Saccharomyces cerevisiae* or *Stenotrophomonas maltophilia,* more preferably Sma_KPR from *Stenotrophomonas maltophilia* NBRC 14161, Cpa_PKR_2 from *Candida parapsilosis* IFO 0708, Cpa_PKR_1 from *Candida parapsilosis* IFO 0708, ADH YJR096w from S. *cerevisiae,* ADH 440 (catalogue # evo 1.1.440, evoXX GmbH, Germany), most preferably Sma_KPR from *Stenotrophomonas maltophilia* NBRC 14161 or Cpa_PKR_2 from *Candida parapsilosis* IFO 0708 or enzymes having equivalent activity of Sma_KPR from *Stenotrophomonas maltophilia* NBRC 14161 or Cpa_PKR_2 from *Candida parapsilosis* IFO 0708 or ADH 440.

As used herein, the term "keto-pantolactone" also includes the respective ring-opened form, i.e. ketopantoic acid.

As used herein, terms like "alcohol dehydrogenase", "reductase" or "enantioselective alcohol dehydrogenase (ADH) enzymes" are used interchangeably herein and define suitable enzymes to be used for the performance of the present invention, i.e. conversion of KPL into pantolactone with a high percentage, i.e. at least about 80%, of (R)-pantolactone.

The terms "conversion", "enantioselective reduction", "biocatalytic process", "enantioselective catalytic reduction" or "enzymatic conversion" are used interchangeably herein and describe the reduction of KPL into (R)-pantolactone catalyzed by the ADHs as defined herein.

As used herein, the term "used in the presence of NADH" or "together with NADH as cofactor" is used interchangeably herein and refers to a reaction wherein NAD is added but the reduced form, i.e. NADH, is actually used by the enzyme as co-factor.

The enzymatic conversion of KPL as defined herein into (R)-pantolactone, as described herein can be performed with isolated enzymes, such as e.g. dehydrogenase enzymes as defined herein, particularly ADHs, such as e.g. ADHs as defined herein originating from *Stenotrophomonas maltophilia, Candida parapsilosis, Saccharomyces cerevisiae,* particularly Sma_KPR, Cpa_PKR_2, Cpa_PKR_1, YJR096w_ADH, that might be expressed in a suitable host cell, such as e.g. yeast or bacteria, preferably Pichia or Escherichia, such as e.g. as heterologous enzymes. Particularly, the enantioselective enzymes as described herein are heterologous expressed in *Escherichia coli.* Isolation of suitable enzymes can be done by known methods, the respective enzymes might be further used in isolated form or in the form of a cell free extract (cfe), powder or liquid formulations, i.e. in immobilized or non-immobilized form. Preferably, the enzymes as defined herein, such as e.g. the reductases from Stenotrophomonas, Candida, Saccharomyces as defined herein, are used as cfe. If the enzymes are used in isolated form, such as e.g. in liquid or immobilized form, they might be either covalently bound or adsorbed, e.g. on epoxy carrier, depending on the supplier. When using immobilized forms, the enzymes might be used several times with more or less the same performance (so-called recycling). Suitable enzymes to be used for the purpose of the present invention might be sourced from commercially available sources, such as e.g. ADH 440 (catalogue # evo 1.1.440, evoXX GmbH, Germany).

The enzymes to be used for the present invention are known as NADPH-dependent alcohol dehydrogenases, i.e., using NADPH as co-factor for their catalytic activity. As used herein, the term "co-factor" and "co-enzyme" are used interchangeably herein.

Thus, the process according to the present invention comprises incubation of KPL in the presence of a suitable enzyme as defined herein at a pH below 7.0, further comprising the presence of a co-factor, particularly wherein the co-factor is selected from NADPH or NADH, preferably selected from NADPH.

The process according to the present invention wherein KPL is enantioselectively converted via action of a reductase together with a co-factor as defined herein at a pH below 7.0 might be combined with an in-situ co-factor regeneration system, such as e.g. co-expression of co-factor regeneration enzymes, including but not limited to glucose dehydrogenase (GDH) converting D-glucose to D-gluconic acid and glucose as substrate, such as e.g. co-expression of *E. coli* GDH. Alternatively, GDH (CAS Number 9028-53-9) is available from various suppliers as known in the art.

In one embodiment, the process according to the present invention using an enzyme as defined herein with the respective co-factor, furthermore optionally combined with co-factor regeneration enzymes, particularly GDH, is performed at a pH below 7.0. Particularly, the process is performed at a pH in the range of about 6.9 to about 5.5, such as e.g. at a pH of about 6.9, 6.8, 6.7, 6.6, 6.5, 6.0, 5.9, 5.8, 5.7, 5.6, 5.5, 5.0, 4.5.

In one embodiment, the process according to the present invention is performed with Sma_KPR from Stenotrophomonas maltophilia NBRC 14161, preferably a polypeptide with at least about 80%, such as e.g. 85, 90, 95, 98 or 100% identity to SEQ ID NO:1, including polynucleotides encoding said polypeptide such as e.g. the polynucleotide of SEQ ID NO:2, at a pH below 7.0, such as e.g. at a pH of about 5.5, resulting in (R)-pantolactone with an e.e. of at least about 85%, particularly comprising a process with NAD as co-factor. The e.e. might be further increased to at least about 97% with NADP as co-factor. Using such process, the yield of converted (R)-pantolactone might be in the range of at least about 87-97%.

In one embodiment, the process according to the present invention is performed with Cpa_PKR_2 from *Candida parapsilosis* IFO 0708, preferably a polypeptide with at least about 80%, such as e.g. 85, 90, 95, 98 or 100% identity to SEQ ID NO:3 including polynucleotides encoding said polypeptide such as e.g. the polynucleotide of SEQ ID NO:4, at a pH below 7.0, such as e.g. at a pH of about 5.5, resulting in (R)-pantolactone with an e.e. of at least about 84%, particularly comprising a process with NAD as co-factor. The e.e. might be further increased to at least about 99% with NADP as co-factor. Using such process, the yield of converted (R)-pantolactone might be in the range of at least about 59-84%.

In another embodiment, the process according to the present invention is performed with Cpa_PKR_1 from *Candida parapsilosis* IFO 0708, preferably a polypeptide with at least about 80%, such as e.g. 85, 90, 95, 98 or 100% identity to SEQ ID NO:5 including polynucleotides encoding said polypeptide such as e.g. the polynucleotide of SEQ ID NO:6, at a pH below 7.0, such as e.g. at a pH of about 5.5, resulting in (R)-pantolactone with an e.e. of at least about 98%, particularly comprising a process with NADP as co-factor. Using such process, the yield of converted (R)-pantolactone might be in the range of at least about 74%.

In another embodiment, the process according to the present invention is performed with YJR096w_ADH from *Saccharomyces cerevisiae,* preferably a polypeptide with at least about 80%, such as e.g. 85, 90, 95, 98 or 100% identity to SEQ ID NO:7 including polynucleotides encoding said polypeptide such as e.g. the polynucleotide of SEQ ID NO:8, at a pH below 7.0, such as e.g. at a pH of about 5.5, resulting in (R)-pantolactone with an e.e. of at least about 97%, particularly comprising a process with NADP as co-factor. Using such process, the yield of converted (R)-pantolactone might be in the range of at least about 67%.

In another embodiment, the process according to the present invention is performed with ADH 440 (EvoXX GmbH, Germany) at a pH below 7.0, such as e.g. at a pH of about 5.5, resulting in (R)-pantolactone with an e.e. of at least about 94%, particularly comprising a process with NAD as co-factor. The e.e. might be further increased to at least about 99% with NADP as co-factor. Using such process, the yield of converted (R)-pantolactone might be in the range of at least about 39-79%.

The "enantiomeric excess" (e.e.) as used herein refers to the degree to which a sample contains one enantiomer (such as e.g. (R)-pantolactone) in higher amount than the other enantiomer (such as e.g. (S)-pantolactone), see Helpfile on selectivity, Uni Graz, 2012: http://biocatalysis.uni-graz.at/enantio/DataFiles/Selectivity-Help.pdf.

The terms "sequence identity", "% identity" are used interchangeable herein. For the purpose of this invention, it is defined here that in order to determine the percentage of sequence identity of two amino acid sequences or of two nucleic acid sequences, the sequences are aligned for optimal comparison purposes. In order to optimize the alignment between the two sequences gaps may be introduced in any of the two sequences that are compared. Such alignment can be carried out over the full length of the sequences being compared.

Alternatively, the alignment may be carried out over a shorter length, for example over about 20, about 50, about 100 or more nucleic acids/bases or amino acids. The sequence identity is the percentage of identical matches between the two sequences over the reported aligned region. The percent sequence identity between two amino acid sequences or between two nucleotide sequences may be determined using the Needleman and Wunsch algorithm for the alignment of two sequences (Needleman, S. B. and Wunsch, C. D. (1970) J. Mol. Biol. 48, 443-453). Both amino acid sequences and nucleotide sequences can be aligned by the algorithm. The Needleman-Wunsch algorithm has been implemented in the computer program NEEDLE. For the purpose of this invention the NEEDLE program from the EMBOSS package was used (version 2.8.0 or higher, EMBOSS: The European Molecular Biology Open Software Suite (2000) Rice, Longden and Bleasby, Trends in Genetics 16, (6) pp276-277, http://emboss.bioinformatics.nl/). For protein sequences EBLOSUM62 is used for the substitution matrix. For nucleotide sequence, EDNAFULL is used. The optional parameters used are a gap-open penalty of 10 and a gap extension penalty of 0.5. The skilled person will appreciate that all these different parameters will yield slightly different results but that the overall percentage identity of two sequences is not significantly altered when using different algorithms.

After alignment by the program NEEDLE as described above the percentage of sequence identity between a query sequence and a sequence of the invention is calculated as follows: number of corresponding positions in the alignment showing an identical amino acid or identical nucleotide in both sequences divided by the total length of the alignment after subtraction of the total number of gaps in the alignment. The identity as defined herein can be obtained from NEEDLE by using the NOBRIEF option and is labeled in the output of the program as "longest identity". If both amino acid sequences which are compared do not differ in any of their amino acids, they are identical or have 100% identity.

As used herein, the term "specific activity" or "activity" with regards to enzymes means its catalytic activity, i.e. its ability to catalyze formation of a product from a given substrate. The specific activity defines the amount of substrate consumed and/or product produced in a given time period and per defined amount of protein at a defined temperature. Typically, specific activity is expressed in µmol substrate consumed or product formed per min per mg of protein. Typically, activity is given per mg of the specific enzyme; if specified otherwise, activity can also be given on mg enzyme preparation (e.g. total protein, total soluble protein etc.). Typically, µmol/min is abbreviated by U (= unit). Therefore, the unit definitions for specific activity of µmol/min/(mg of protein) or U/(mg of protein) are used interchangeably throughout this document. An enzyme is active, if it performs its catalytic activity in vivo, i.e. within the host cell as defined herein or within an in vitro system in the presence of a suitable substrate. The skilled person knows how to measure enzyme activity, in particular activity of ADHs as defined herein, such as in particular KPL-reductase activity. Analytical methods to evaluate the capability of a suitable enzyme as defined herein for formation of (R)-pantolactone from conversion of KPL as defined herein are known in the art.

The present invention is directed to a process as defined herein for enantioselective reduction of KPL, wherein the KPL is enzymatically converted into (R)-pantolactone at a pH below 7.0, particularly pH of about 5.5, with an e.e. in the range of at least about 59-99%, and a yield of (R)-pantolactone of at least about 59%, depending on the used enzyme and on the used co-factor to be added.

In one aspect of the present invention, a host cell comprising and expressing an enantioselective enzyme as defined herein, is used in a process for production of (R)-pantolactone from conversion of KPL as defined herein. The host cell comprising the enzyme may be cultured in an aqueous medium supplemented with appropriate nutrients under aerobic or anaerobic conditions and as known by the skilled person for the respective host cells. Optionally, such cultivation is in the presence of proteins and/or co-factors involved in transfer of electrons, as known in the art, particularly co-factor selected from NAD(P)H or NADH, preferably NADH. The cultivation/growth of the host cell may be conducted in batch, fed-batch, semi-continuous or continuous mode. (R)-pantolactone might be isolated and/or optionally further purified.

In one embodiment of the invention the conversion as described herein is carried out using an immobilized enzyme for enantioselective conversion of KPL into (R)-pantolactone. Such process might be performed under conditions known in the art, e.g. such as in an aqueous medium or under conditions mentioned herein. Particularly, the process might be carried out at pH about 5.5, using NADP and GDH/glucose as in-situ co-factor regeneration system.

With regards to the present invention, it is understood that organisms, such as e.g. animals, microorganisms, fungi, algae or plants also include synonyms or basonyms of such species having the same physiological properties, as defined by the International Code of Zoological Nomenclature, International Code of Nomenclature of Prokaryotes or the International Code of Nomenclature for algae, fungi, and plants (Melbourne Code).

The process as described herein might be carried out at temperatures of about 21°C to about 45°C, such as e.g. between about 21°C to about 40°C, particularly between about 21°C to about 35°C, preferably between about 25°C to about 31°C, more preferably about 28°C.

The following examples are illustrative only and are not intended to limit the scope of the invention in any way.

### Examples

### Example 1: General methods and media

All basic molecular biology and DNA manipulation procedures described herein were generally performed according to Sambrook et al. (1989. Molecular Cloning: A Laboratory Manual. Cold Spring Harbor Laboratory Press: New York) or Ausubel et al. (1998. Current Protocols in Molecular Biology. Wiley: New York). Media used for protein production are listed in Table 1.

**Table 1: Media composition.**

| Medium | Components |
|---|---|
| LB | 5 g/l yeast extract |
| | 10 g/l tryptone |
| | 5 g/l NaCl |
| 2xTY | 10 g/l yeast extract (final) |
| | 16 g/l tryptone |
| | 5 g/l NaCl |

### Example 2: Expression of enzymes in Escherichia coli

Gene encoding sequences 1, 3, 5, 7, were ordered codon-optimized for expression in E. coli as synthetic DNA at ATUM (Newark, California, US). All constructs were used for transformation of E. coli RV308ΔrhaBAD. Transformations were done as follows:
Competent cells were thawed on ice. Subsequently, 1 µl plasmid DNA was added to the cells and the mix was incubated for 5 minutes on ice. After a heat shock treatment (60 sec, 42°C) cells were immediately placed on ice again for 5 minutes. 300 µl SOC medium (Catalogue # 15544034, Thermo Fisher Scientific, USA) were added and the cells were incubated for 1-2 hours at 37°C and gentle shaking (300 rpm). Subsequently, 50 µl of the reaction mix was plated on LB medium containing 50 µg/ml neomycin. Alternatively, the full reaction mix was centrifuged (2 min, 6000 rpm) and the cell pellet was resuspended in 100 µl SOC medium and completely plated on the selective agar plates. Selection plates were incubated overnight at 37°C.

Recombinant strains were cultivated for ADH expression as follows: 20 ml of LB medium containing 100 mg/l neomycin were inoculated with the glycerol stock of the respective clone. The culture was incubated over-night at 28°C and with shaking at 110 rpm. The next day, 100 ml 2xTY medium containing 100 mg/l neomycin in a 500 ml baffled Erlenmeyer flask were inoculated with 1 ml of the preculture (initial OD620 ∼0.05). The culture was incubated on an orbital shaker (180 rpm) at 28°C. When the OD620 reached ∼0.5, the protein expression was induced by addition of 100 µl 20% (v/v) L-rhamnose (final concentration 0.02%). The cultivation of the strains was continued over-night (180 rpm, 28°C). On the next day, cells were harvested by centrifugation at 4°C for 15 min and 5000 rpm. Cell pellets were stored at -20°C.

For further studies cell free extract was prepared. Wet cells were resuspended in 200 vol% of 100 mM potassium phosphate buffer pH 7.0 with respect to their wet weight. The cell suspension was sonicated on ice until the OD620 of the suspension was below 10% of the starting value. Cell debris was subsequently removed by centrifugation (4°C, 5000 rpm for 10 min). The clear supernatant (cell free extract, CFE) was stored until further use at -20°C.

### Example 3: Enantioselective reduction of KPL into (R)-pantolactone at pH 5.5

Formation of (R)-pantolactone was tested with different enzymes using both NAD⁺ or NADP⁺ as co-factor at constant pH 5.5. Reactions on a 10 ml scale were performed with 100 mM KPL, MgCL₂ (1mM final conc.) ZnSO₄ (1mM final concentration), 5% (vol/vol) enzyme (CFE; see Ex. 2), 150 mM (final conc.) D-glucose & 20 U/ml (final conc.) of GDH (Codexis GDH-105). Co-factors (0.7 mM final conc.) were added. Incubations were done at 28°C, shaken (stirrer speed 10) for 1 -3 hours.

250 µl sample was acidified with 25 µl of HCl (37%), heated at 80°C for 15 min and extracted with 900 µl ethyl acetate with internal standard (anisole, ca. 1 mg/ml). After 10 min of shaking, the phases separated within 10 min and a sample of the ethyl acetate layer was taken for analysis.

Measurements were carried out on an Agilent HP 6890 gas chromatograph equipped with hot-split injector, autosampler and FID detector. Column used was Chirasil DEX CB, dimensions 25 m x 250 µm x 0.25 µm film thickness. The isothermal temperature was 125°C during 12 minutes. As a carrier gas Helium with a flow of 1.5 ml/min was used. Injection volume was 1 µl, with a split ratio of 1 : 30 at a hot split injection at 220°C. A flame ionisation detector was used at 250°C, with a hydrogen flow of 40 mL/min, an air flow of 450 mL/min and a make-up flow of 30 mL/min.

The results for ketopantolactone reductase from S. maltophila,polyketone reductase from C. parapsilosis (Cpa_PKR_2 and Cpa_PKR_1) or ADH from S. cerevisiae (YJR096w_ADH) compared to ADH 440 are shown in Table 2 below.

**Table 2A: (R)-selectivity of selected enzymes and NADP as co-factor. "ee R-PL" means ee of (R)-pantolactone measured at the end, "yield R-PL" means the percentage of (R)-pantolactone based on mol. All enzymes are used as CFE, except for ADH 440 which is in powder form. The bio blank contains CFE of E. coli RV308ΔrhaBAD (no plasmid). The chemical blank contains all components of the reaction but no CFE. For more details, see text.**

| Enzyme | Mass balance (mol based) | ee R-PL | Yield R-PL |
|---|---|---|---|
| Sma_KPR | 98 | 97 | 97 |
| Cpa_PKR_2 | 99 | 99 | 84 |
| Cpa_PKR_1 | 98 | 98 | 74 |
| YJR096w_ADH | 89 | 97 | 67 |
| ADH 440 | 96 | 99 | 79 |
| Bio blank | 98 | 8 | 10 |
| Chemical blank | 101 | 54 | 1 |

**Table 2B: (R)-selectivity of selected enzymes and NAD as co-factor. "ee R-PL" means ee of (R)-pantolactone measured at the end, "yield R-PL" means the percentage of (R)-pantolactone based on mol. All enzymes are used as CFE, except for ADH 440 which is in powder form. For more details, see text or legend to Table 2A.**

| Enzyme | Mass balance (mol based) | ee R-PL | Yield R-PL |
|---|---|---|---|
| Sma_KPR | 97 | 85 | 87 |
| Cpa_PKR_2 | 98 | 84 | 59 |
| Cpa_PKR_1 | 99 | 27 | 23 |
| YJR096w_ADH | 97 | 47 | 28 |
| ADH 440 | 96 | 94 | 39 |
| Bio blank | 98 | -35 | 5 |
| Chemical blank | n.a. | n.a. | n.a. |

## Claims

1. Process for enantioselective conversion of keto-pantolactone into R-pantolactone, comprising incubation of keto-pantolactone in the presence of a NADPH dependent reducing enzyme at a pH of below 7.0.

2. Process according to claim 1, wherein the pH is in the range of about 6.9 to about 5.5, preferably in the range of about 5.5.

3. Process according to claim 1 or 2, wherein the reducing enzyme is an alcohol dehydrogenase originating from Candida, Stenotrophomonas or Yarrowia, particularly selected from *Candida parapsilosis, Saccharomyces cerevisiae* or *Stenotrophomonas maltophilia,* preferably Sma_KPR from *Stenotrophomonas maltophilia* NBRC 14161, Cpa_PKR_2 from *Candida parapsilosis* IFO 0708, Cpa_PKR_1 from *Candida parapsilosis* IFO 0708, ADH YJR096w from S. cerevisiae, most preferably Sma_KPR from *Stenotrophomonas maltophilia* NBRC 14161 with at least about 80% identity to SEQ ID NO:1 or Cpa_PKR_2 from *Candida parapsilosis* IFO 0708 with at least about 80% identity to SEQ ID NO:3 or Cpa_PKR_1 from *Candida parapsilosis* IFO 0708 with at least about 80% identity to SEQ ID NO:5, or ADH YJR096w from *S*. *cerevisiae* with at least about 80% identity to SEQ ID NO:7.

4. Process according to claim 3, wherein the enzyme is used as a recombinant whole cell biocatalyst or as cell free extract.

5. Process according to claim 1 or 2, wherein the reducing enzyme is a commercially available NADPH-dependent alcohol dehydrogenase, particularly ADH 440 (catalogue # evo 1.1.440, evoXX GmbH, Germany).

6. Process according to any one of claims 1 to 5, wherein the reducing enzyme is co-incubated with a co-factor selected from NADPH or NADH, preferably NADPH.

7. Process according to any one of claims 1 to 6, further comprising an in-situ co-factor regeneration system, particularly comprising glucose dehydrogenase and glucose.

8. Process for production of (R)-pantolactone with e.e. of at least about 97%, comprising enantioselective reduction of keto-pantolactone in the presence of an enantioselective alcohol dehydrogenase (ADH), particularly an ADH enzyme selected from *Candida parapsilosis, Saccharomyces cerevisiae* or *Stenotrophomonas maltophilia,* preferably Sma_KPR from *Stenotrophomonas maltophilia* NBRC 14161, Cpa_PKR_2 from *Candida parapsilosis* IFO 0708, Cpa_PKR_1 from *Candida parapsilosis* IFO 0708, ADH YJR096w from *S*. *cerevisiae,* or ADH 440 (evoXX), most preferably an enzyme with at least about 80% identity to SEQ ID NOs: 1, 3, 5, or 7, together with NADPH as co-factor.

9. Process for production of (R)-pantolactone with e.e. of at least about 94%, comprising enantioselective reduction of keto-pantolactone in the presence of the enantioselective alcohol dehydrogenase ADH 440 (catalogue # evo 1.1.440, evoXX GmbH, Germany) together with NADPH or NADH as co-factor.

10. Process according to claim 8, wherein the yield of (R)-pantolactone is at least about 67%.


SEQUENCE LISTING

<110>  DSM IP Assets B.V.

<120>  Enantioselective production of (R)-pantolactone

<130>  Case 33807

<160>  8

<170>  PatentIn version 3.5

<210>  1
<211>  258
<212>  PRT
<213>  Stenotrophomonas maltophilia

<400>  1

Met Thr Gln Gln Arg Trp Arg Leu Asp Gly Gln Thr Ala Leu Ile Thr
1               5                   10                  15

Gly Ala Ser Ala Gly Ile Gly Leu Ala Ile Ala His Glu Leu Ala Gly
            20                  25                  30

Phe Gly Ala Asp Leu Met Ile Val Gly Arg Asp Ile Asp Met Leu Glu
        35                  40                  45

Thr Ala Arg Asp Glu Leu Leu Asp Val Tyr Pro Gln Met Gln Val His
    50                  55                  60

Ala Leu Ala Ala Asp Val Ser Asp Asp Glu Asp Arg Arg Gln Ile Leu
65                  70                  75                  80

Asp Trp Val Glu Asp His Ser Asp Gly Leu His Ile Leu Val Asn Asn
                85                  90                  95

Ala Gly Gly Asn Val Thr Lys Ala Ala Thr Glu Tyr Ser Glu Asp Glu
            100                 105                 110

Trp Arg Lys Ile Phe Glu Thr Asn Leu Phe Ser Ala Phe Glu Leu Ser
        115                 120                 125

Arg Tyr Ala His Pro Leu Leu Ala Arg His Ala Ser Ser Ser Ile Val
    130                 135                 140

Asn Val Gly Ser Val Ser Gly Leu Thr His Val Arg Ser Gly Val Val
145                 150                 155                 160

Tyr Gly Met Ser Lys Ala Ala Met His Gln Met Thr Arg Asn Leu Ala
                165                 170                 175

Val Glu Trp Ala Glu Asp Gly Ile Arg Val Asn Ala Val Ala Pro Trp
            180                 185                 190

Tyr Ile Arg Thr Arg Arg Thr Ser Gly Pro Leu Ser Asp Pro Asp Tyr
        195                 200                 205

Tyr Glu Glu Val Ile Asn Arg Thr Pro Met Arg Arg Ile Gly Glu Pro
    210                 215                 220

Glu Glu Val Ala Ala Ala Val Gly Phe Leu Cys Leu Pro Ala Ala Ser
225                 230                 235                 240

Tyr Val Thr Gly Glu Cys Ile Ala Val Asp Gly Gly Phe Leu Arg Tyr
                245                 250                 255

Gly Phe

<210>  2
<211>  777
<212>  DNA
<213>  Stenotrophomonas maltophilia

<400>  2
atgacgcaac aacgctggcg cttagacggt caaactgcat taattacggg tgcttcggcc      60

ggtatcggcc tggcgattgc gcacgaactg gcgggcttcg gtgcggatct gatgattgtg     120

ggtcgtgata ttgatatgct ggagactgcc cgtgacgaac tgcttgacgt ttaccctcag     180

atgcaagtgc acgcgttggc ggctgacgtg agcgatgacg aagatcgccg ccagatcctg     240

gactgggtcg aggatcacag cgacggtctg catattctgg taaacaacgc cggtggcaac     300

gtgaccaaag ccgcaaccga gtattctgaa gatgagtggc gtaagatctt tgaaacgaat     360

ctgttcagcg cgtttgagct gtcccgttat gcacacccgt tgctggcccg tcatgcgagc     420

agcagcatcg tcaatgttgg cagcgtttcc ggtctgaccc acgtccgcag cggtgtcgtg     480

tacggtatga gcaaagctgc gatgcatcag atgacccgta acctggcagt cgagtgggcg     540

gaagatggca ttcgtgtgaa tgccgttgcg ccgtggtata tccgtacccg ccgtaccagc     600

ggtccgctgt ctgacccgga ctactacgaa gaggttatca atagaacgcc aatgcgccgt     660

attggcgagc cggaagaggt cgcggcagcg gttggttttc tgtgcctgcc ggctgcaagc     720

tacgtgaccg gtgagtgtat cgcagttgac ggcggcttct tgcgttatgg tttctaa        777

<210>  3
<211>  307
<212>  PRT
<213>  Candida parapsilosis

<400>  3

Met Thr Gln Ser Asn Leu Leu Pro Lys Thr Phe Arg Thr Lys Ser Gly
1               5                   10                  15

Lys Glu Ile Ser Ile Ala Leu Gly Thr Gly Thr Lys Trp Lys Gln Ala
            20                  25                  30

Gln Thr Ile Asn Asp Val Ser Thr Glu Leu Val Asp Asn Ile Leu Leu
        35                  40                  45

Gly Leu Lys Leu Gly Phe Arg His Ile Asp Thr Ala Glu Ala Tyr Asn
    50                  55                  60

Thr Gln Lys Glu Val Gly Glu Ala Leu Lys Arg Thr Asp Val Pro Arg
65                  70                  75                  80

Glu Asp Ile Trp Val Thr Thr Lys Tyr Ser Pro Gly Trp Gly Ser Ile
                85                  90                  95

Lys Ala Tyr Ser Lys Ser Pro Ser Asp Ser Ile Asp Lys Ala Leu Ala
            100                 105                 110

Gln Leu Gly Val Asp Tyr Val Asp Leu Phe Leu Ile His Ser Pro Phe
        115                 120                 125

Phe Thr Thr Glu Gln Thr His Gly Tyr Thr Leu Glu Gln Ala Trp Glu
    130                 135                 140

Ala Leu Val Glu Ala Lys Lys Ala Gly Lys Val Arg Glu Ile Gly Ile
145                 150                 155                 160

Ser Asn Ala Ala Ile Pro His Leu Glu Lys Leu Phe Ala Ala Ser Pro
                165                 170                 175

Ser Pro Glu Tyr Tyr Pro Val Val Asn Gln Ile Glu Phe His Pro Phe
            180                 185                 190

Leu Gln Asn Gln Ser Lys Asn Ile Val Arg Phe Cys Gln Glu His Gly
        195                 200                 205

Ile Leu Val Glu Ala Phe Ser Pro Leu Ala Pro Leu Ala Arg Val Glu
    210                 215                 220

Thr Asn Ala Leu Ala Glu Thr Leu Lys Arg Leu Ala Glu Lys Tyr Lys
225                 230                 235                 240

Lys Thr Glu Ala Gln Val Leu Leu Arg Tyr Thr Leu Gln Arg Gly Ile
                245                 250                 255

Leu Pro Val Thr Thr Ser Ser Lys Glu Ser Arg Leu Lys Glu Ser Leu
            260                 265                 270

Asn Leu Phe Asp Phe Glu Leu Thr Asp Glu Glu Val Asn Glu Ile Asn
        275                 280                 285

Lys Ile Gly Asp Ala Asn Pro Tyr Arg Ala Phe Phe His Glu Gln Phe
    290                 295                 300

Lys Asp Leu
305

<210>  4
<211>  924
<212>  DNA
<213>  Candida parapsilosis

<400>  4
atgacccaaa gcaatttact gccaaaaacg ttccgtacca agtctggcaa agaaattagc      60

attgctctgg gcacgggcac caagtggaaa caagcacaga cgatcaacga cgttagcacc     120

gaactggttg acaatatcct tctgggtctg aaactgggtt tccgccacat cgatacggcc     180

gaggcttaca atacgcaaaa agaggtcggt gaggctctga agcgtaccga cgttccgcgc     240

gaagatatct gggtgactac caagtatagc ccgggttggg gtagcatcaa ggcatatagc     300

aaaagcccga gcgactctat cgataaggcc ttggcgcaac tgggcgtgga ttacgtggac     360

ctctttctga ttcattcacc gtttttcacg accgagcaga cccacggtta cacgctggag     420

caagcctggg aagcgctggt tgaggcgaaa aaagccggca aggtccgcga gatcggtatt     480

agcaacgcag cgattcctca cctggaaaag ctgttcgccg cgagcccaag cccggagtac     540

tatccggtcg ttaaccagat cgagtttcac ccgtttttgc aaaaccagtc caagaatatt     600

gtccgttttt gccaggaaca tggcattctg gtggaagcgt tcagcccgct ggcaccgctg     660

gcgcgtgtgg aaacgaatgc gctggcggag actctgaaac gtttggcaga gaagtacaag     720

aaaaccgaag cacaggtcct gctgcgttac accctgcagc gtggtatttt gccggttacc     780

acctccagca aagaatcccg tttgaaagaa tcgctgaacc tgttcgattt cgagctgacc     840

gatgaagagg tgaacgaaat caataagatt ggtgacgcga acccgtatcg cgcgttcttt     900

catgaacagt ttaaagacct gtaa                                            924

<210>  5
<211>  304
<212>  PRT
<213>  Candida parapsilosis

<400>  5

Met Ser Leu Ala Gly Lys Glu Phe Lys Leu Ser Asn Gly Asn Lys Ile
1               5                   10                  15

Pro Ala Val Ala Phe Gly Thr Gly Thr Lys Tyr Phe Lys Arg Gly His
            20                  25                  30

Asn Asp Leu Asp Lys Gln Leu Ile Gly Thr Leu Glu Leu Ala Leu Arg
        35                  40                  45

Ser Gly Phe Arg His Ile Asp Gly Ala Glu Ile Tyr Gly Thr Asn Lys
    50                  55                  60

Glu Ile Gly Ile Ala Leu Lys Asn Val Gly Leu Asn Arg Lys Asp Val
65                  70                  75                  80

Phe Ile Thr Asp Lys Tyr Asn Ser Gly Asn His Thr Tyr Asp Gly Lys
                85                  90                  95

His Ser Lys His Gln Asn Pro Tyr Asn Ala Leu Lys Ala Asp Leu Glu
            100                 105                 110

Asp Leu Gly Leu Glu Tyr Val Asp Leu Tyr Leu Ile His Phe Pro Tyr
        115                 120                 125

Ile Ser Glu Lys Ser His Gly Phe Asp Leu Val Glu Ala Trp Arg Tyr
    130                 135                 140

Leu Glu Arg Ala Lys Asn Glu Gly Leu Ala Arg Asn Ile Gly Val Ser
145                 150                 155                 160

Asn Phe Thr Ile Glu Asn Leu Lys Ser Ile Leu Asp Ala Asn Thr Asp
                165                 170                 175

Ser Ile Pro Val Val Asn Gln Ile Glu Phe Ser Ala Tyr Leu Gln Asp
            180                 185                 190

Gln Thr Pro Gly Ile Val Glu Tyr Ser Gln Gln Gln Gly Ile Leu Ile
        195                 200                 205

Glu Ala Tyr Gly Pro Leu Gly Pro Ile Thr Gln Gly Arg Pro Gly Pro
    210                 215                 220

Leu Asp Lys Val Leu Ser Lys Leu Ser Glu Lys Tyr Lys Arg Asn Glu
225                 230                 235                 240

Gly Gln Ile Leu Leu Arg Trp Val Leu Gln Arg Gly Ile Leu Pro Ile
                245                 250                 255

Thr Thr Thr Ser Lys Glu Glu Arg Ile Asn Asp Val Leu Glu Ile Phe
            260                 265                 270

Asp Phe Glu Leu Asp Lys Glu Asp Glu Asp Gln Ile Thr Lys Val Gly
        275                 280                 285

Lys Glu Lys Thr Leu Arg Gln Phe Ser Lys Glu Tyr Ser Lys Tyr Asp
    290                 295                 300

<210>  6
<211>  915
<212>  DNA
<213>  Candida parapsilosis

<400>  6
atgagcttgg ccggcaaaga attcaaactg tctaatggta ataagatccc agcggttgcg      60

tttggtacgg gcactaaata ctttaagcgc ggtcataacg acctggataa gcaattgatt     120

ggcacgctgg aattggctct gcgcagcggt ttccgtcaca tcgatggtgc agagatctac     180

ggtaccaaca aagaaatcgg tattgcactg aaaaatgttg gcctgaatcg taaagatgtg     240

ttcattaccg ataagtataa cagcggtaat catacctacg acggtaagca ctccaagcac     300

cagaatccgt ataacgcgct gaaagccgac ctggaagatt tgggtcttga atacgtcgac     360

ctctatctga ttcatttccc gtacatcagc gagaagtccc acggtttcga tctggtcgag     420

gcgtggcgct acttagagcg tgctaaaaac gagggtctgg cccgcaatat tggcgtgagc     480

aactttacca ttgagaatct gaagtcgatt ctggacgcaa acaccgacag catcccggtc     540

gtcaaccaga tcgagtttag cgcgtatttg caagatcaga cgcctggcat tgtggagtac     600

agccagcaac agggcatcct gattgaagcg tatggtccgc tgggcccgat tacccagggc     660

cgtccgggtc cgctggacaa agtgctgagc aagctgagcg agaagtacaa gcgtaacgag     720

ggtcaaattc tgctgcgttg ggtgctgcag cgtggtatcc tgccgatcac cacgaccagc     780

aaagaggaac gtatcaacga cgttctggaa atcttcgact ttgagctgga caaggaagat     840

gaagatcaaa ttaccaaggt tggcaaagaa aaaacgctgc gccaattcag caaagaatat     900

tctaaatacg actaa                                                      915

<210>  7
<211>  282
<212>  PRT
<213>  Saccharomyces cerevisiae

<400>  7

Met Val Pro Lys Phe Tyr Lys Leu Ser Asn Gly Phe Lys Ile Pro Ser
1               5                   10                  15

Ile Ala Leu Gly Thr Tyr Asp Ile Pro Arg Ser Gln Thr Ala Glu Ile
            20                  25                  30
Val Tyr Glu Gly Val Lys Cys Gly Tyr Arg His Phe Asp Thr Ala Val
        35                  40                  45
Leu Tyr Gly Asn Glu Lys Glu Val Gly Asp Gly Ile Ile Lys Trp Leu
    50                  55                  60
Asn Glu Asp Pro Gly Asn His Lys Arg Glu Glu Ile Phe Tyr Thr Thr
65                  70                  75                  80
Lys Leu Trp Asn Ser Gln Asn Gly Tyr Lys Arg Ala Lys Ala Ala Ile
                85                  90                  95
Arg Gln Cys Leu Asn Glu Val Ser Gly Leu Gln Tyr Ile Asp Leu Leu
            100                 105                 110
Leu Ile His Ser Pro Leu Glu Gly Ser Lys Leu Arg Leu Glu Thr Trp
        115                 120                 125
Arg Ala Met Gln Glu Ala Val Asp Glu Gly Leu Val Lys Ser Ile Gly
    130                 135                 140
Val Ser Asn Tyr Gly Lys Lys His Ile Asp Glu Leu Leu Asn Trp Pro
145                 150                 155                 160
Glu Leu Lys His Lys Pro Val Val Asn Gln Ile Glu Ile Ser Pro Trp
                165                 170                 175
Ile Met Arg Gln Glu Leu Ala Asp Tyr Cys Lys Ser Lys Gly Leu Val
            180                 185                 190
Val Glu Ala Phe Ala Pro Leu Cys His Gly Tyr Lys Met Thr Asn Pro
        195                 200                 205
Asp Leu Leu Lys Val Cys Lys Glu Val Asp Arg Asn Pro Gly Gln Val
    210                 215                 220
Leu Ile Arg Trp Ser Leu Gln His Gly Tyr Leu Pro Leu Pro Lys Thr
225                 230                 235                 240
Lys Thr Val Lys Arg Leu Glu Gly Asn Leu Ala Ala Tyr Asn Phe Glu
                245                 250                 255
Leu Ser Asp Glu Gln Met Lys Phe Leu Asp His Pro Asp Ala Tyr Glu
            260                 265                 270

Pro Thr Asp Trp Glu Cys Thr Asp Ala Pro
        275                 280

<210>  8
<211>  849
<212>  DNA
<213>  Saccharomyces cerevisiae

<400>  8
atggtgccga agttctataa actgtccaat ggttttaaaa tcccatctat tgcgctgggt      60

acctatgata ttccgcgctc ccagactgcg gagatcgtat acgaaggcgt aaaatgtggc     120

taccgccatt ttgatactgc ggttctgtat ggtaacgaga aagaagtcgg tgacggcatt     180

atcaaatggc tgaacgagga cccgggtaac cataaacgtg aagaaatctt ctacaccacg     240

aaactgtgga actctcagaa tggctataaa cgcgctaaag cggctattcg tcagtgtctg     300

aacgaagttt ctggcctgca gtacatcgat ctgctgctga ttcattctcc tctggaaggc     360

tctaagctgc gtctggaaac ttggcgtgca atgcaggagg cagtagacga aggtctggta     420

aaatccattg gcgtttccaa ctacggtaag aagcacattg atgaactgct gaactggccg     480

gagctgaaac ataaaccggt tgtgaaccag atcgaaatct ctccgtggat catgcgccag     540

gaactggctg attactgtaa aagcaaaggc ctggttgtcg aagcatttgc gccgctgtgc     600

catggctata aaatgaccaa tcctgacctg ctgaaggtgt gtaaagaagt tgaccgcaac     660

cctggtcagg ttctgattcg ttggagcctg cagcacggtt acctgcctct gccaaagacg     720

aaaaccgtca aacgtctgga aggtaacctg gctgcctaca acttcgagct gtctgatgaa     780

cagatgaaat tcctggacca cccggacgcg tatgagccta ctgattggga gtgcaccgac     840

gcaccgtaa                                                             849